(19) Europäisches Patentamt
European Patent Office
Office européen des brevets

(11) Veröffentlichungsnummer: **0 127 121**
**A1**

# (12) EUROPÄISCHE PATENTANMELDUNG

(21) Anmeldenummer: 84105868.8

(22) Anmeldetag: 23.05.84

(51) Int. Cl.³: C 07 D 333/32
C 07 C 149/20

(30) Priorität: 24.05.83 DE 3318775

(43) Veröffentlichungstag der Anmeldung:
05.12.84 Patentblatt 84/49

(84) Benannte Vertragsstaaten:
BE CH DE FR GB IT LI NL

(71) Anmelder: CASSELLA Aktiengesellschaft
Hanauer Landstrasse 526
D-6000 Frankfurt am Main 61(DE)

(72) Erfinder: Schuster, Karl-Heinz, Dr.
Adalbert Stifter Strasse 35
D-6457 Maintal 2(DE)

(72) Erfinder: Kühlein, Klaus, Dr.
Fasanenstrasse 41
D-6233 Kelkheim(DE)

(72) Erfinder: Mix, Konrad, Dr.
Fuldaer Strasse 34
D-6000 Frankfurt/Main 61(DE)

(72) Erfinder: Müller, Rolf, Dr.
Dornbachstrasse 3
D-6367 Karben 4(DE)

(74) Vertreter: Urbach, Hans-Georg, Dr. et al,
Hanauer Landstrasse 526
D-6000 Frankfurt am Main 61(DE)

## (54) Verfahren zur Herstellung von Tetrahydro-thiophen-3-on.

(57) Verfahren zur Herstellung von Tetrahydro-thiophen-3-on der Formel

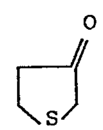

bei dem Thioglycolsäure mit Acrylsäure in Abwesenheit von Lösungsmitteln in Gegenwart eines Knoevenagel-Katalysators und/oder in Gegenwart einer reaktionsfähigen Erdalkaliverbindung miteinander umgesetzt werden und das Additionsprodukt in einem polaren organischen Lösungsmittel in Gegenwart einer reaktionsfähigen Erdalkaliverbindung unter Abdestillieren der flüchtigen Pyrolyseprodukte auf 180 bis 250°C erwärmt wird. Die beiden Reaktionsschritte des Verfahrens können auch zur Herstellung von 3-Thia-adipinsäure, bzw. von Tetrahydro-thiophen-3-on aus 3-Thia-adipinsäure benutzt werden.

EP 0 127 121 A1

## Verfahren zur Herstellung von Tetrahydro-thiophen-3-on

Tetrahydro-thiophen-3-on ist eine wertvolle organische Schlüsselsubstanz, die den synthetischen Zugang zu zahlreichen Derivaten des Thiophens eröffnet. So ist es beispielsweise bekannt, (Recueil des Travaux Chimiques des Pays-Bas 83 (1964), S. 1160 ff.) über Enamine des Tetrahydro-thiophen-3-ons Aminothiophen- und Aminotetrahydrothiophen-Derivate herzustellen. Es ist daher bereits vielfach versucht worden, das Tetrahydro-thiophen-3-on herzustellen, und es wurden mit einigen Variationen im wesentlichen zwei Wege beschritten. Eine größere Anzahl von Publikationen betrifft das Syntheseschema

$ROOC-CH_2-S-CH_2-CH_2-COOR$ (1)

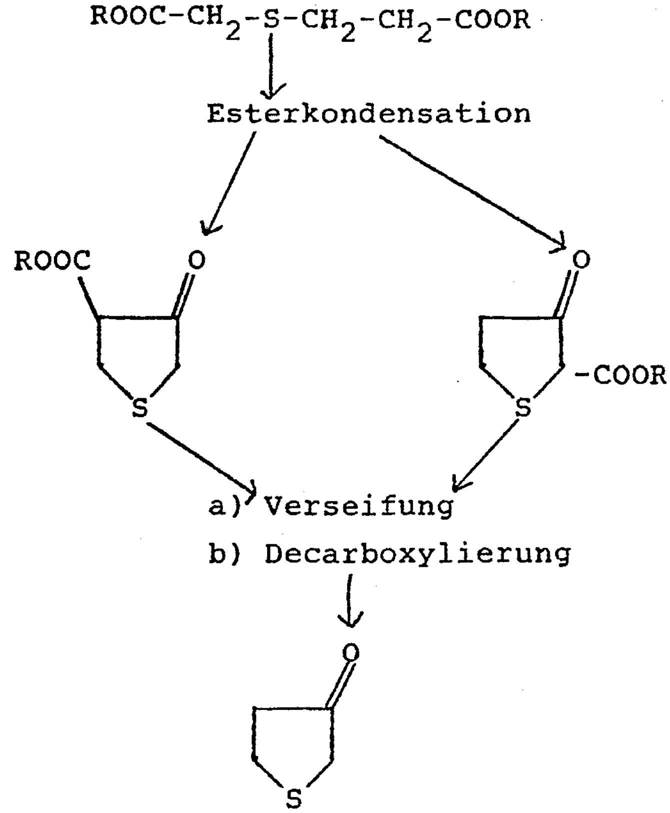


(2)

Stellvertretend für weitere andere Veröffentlichungen seien genannt: J. Amer. Chem. Soc. 66 (1944), S. 847 und 849; 68 (1946), S. 2229; 79 (1957), S. 1972. Der dort beschriebene Prozeß verläuft über drei Stufen, nämlich die Esterkonden

sation des Thiaadipinsäure-diesters (1) zu den isomeren Tetrahydro-thiophenon-carbonsäureestern (2), die Verseifung dieser Ester und die Decarboxylierung der freien Karbonsäuren. Vorgeschaltet ist natürlich die Gewinnung des Ausgangsmaterials (1), die beispielsweise nach J. Amer. Chem. Soc. 68 (1946), S. 2229 durch Umsetzung von Ethyl-ß-brompropionat mit Natrium-thioglycolat und anschließende Veresterung der freien Carboxylgruppe in nicht angegebener Ausbeute oder durch Addition von Methyl-thioglycolat an Methylacrylat in Gegenwart von Piperidin in einer Ausbeute vom 78 % d. Th. erfolgte. Abgesehen von den zahlreichen Zwischenstufen und den damit verbundenen wiederholten Aufarbeitungs- und Reinigungsoperationen ist die Gesamtausbeute der Herstellung von Tetrahydrothiophen-3-on ausgehend von Thioglycolsäure- und Acrylsäurederivaten nach diesem bekannten Reaktionsschema unbefriedigend und für eine Umsetzung in technische Maßstäbe ungeeignet.

Einen weiteren Zugang zu Tetrahydro-thiophen-3-on stellt die Cyclisierung von 3-Thia-adipinsäure in Gegenwart von Bariumhydroxyd dar, die in Recueil des Travaux Chimiques des Pays-Bas 83 (1964), S. 1160 ff. beschrieben ist. Diese Methode liefert nach den dortigen Angaben eine Ausbeute von 44 % d. Th. neben zähen, teerartigen Neben- und Zersetzungsprodukten, die die Reindarstellung der erwünschten Substanz erheblich behindern.

Die direkte Cyclisierung, die gegenüber den Esterkondensations-Verfahren den Vorteil der Einstufigkeit hat, benötigt als Ausgangsmaterial die 3-Thia-adipinsäure. Diese Verbindung kann beispielsweise nach J. Amer. Chem. Soc. 68 (1946) S. 2229 ff. durch Verseifung des in guter Ausbeute aus ß-Brompropionsäureester und Di-natrium-thioglycolat herstellbaren 3-Thia-adipinsäure-monoesters erhalten werden. Der Umweg über den Ester kann auch durch direkte Addition von Thioglycolsäure an Acrylsäure vermieden werden, jedoch ist

nach den Angaben der Literatur dieser Weg nicht unproblematisch. Nach den Angaben von Schöberl, Ber. dtsch. Chem. Ges. 80 (1947), S. 379 ff., läßt sich diese Addition nur bei pH-Werten >7 jedoch nicht im stark alkalischen Bereich durchführen, und es muß unbedingt ein erheblicher Acrylsäure-Überschuß eingesetzt werden (100 % Überschuß). In späteren Veröffentlichungen des gleichen Autors (Schöberl und Lange, Liebigs Ann. Chem. 599 (1956), S. 140 ff.) wird zwar gezeigt, daß Thioglycolsäure sich auch im stark sauren und stark alkalischen wäßrigen Medium an Acrylsäure addieren läßt, jedoch ist die Ausbeute in dem auf S. 154 beschriebenen Ausführungsbeispiel mit 41 % nur mäßig, und der hierbei eingesetzte Acrylsäure-Überschuß von 300 % (!) stellt einen erheblichen Nachteil dar, der eine Übertragung dieser Methode in die Technik nicht aussichtsreich erscheinen läßt. Reaktionsbedingungen, unter denen sich bessere Ausbeuten, z.B. die der Tabelle auf S. 154 der genannten Literaturstelle, erzielen ließen, sind nicht ersichtlich.

Es wurde nun gefunden, daß sich trotz der genannten Nachteile der bisher beschriebenen Umsetzungen ein Syntheseweg ausgehend von Thioglycolsäure und Acrylsäure zum Tetrahydro-thiophen-3-on bietet, der den Vorteil hat, sich auf technische Dimensionen übertragen zu lassen.

Das erfindungsgemäße Verfahren zur Herstellung von Tetrahydro-thiophen-3-on besteht darin, daß man Thioglycolsäure mit Acrylsäure im Molverhältnis von 1 : 0,7 bis 1 : 1,5 , vorzugsweise von 1 : 1, in Abwesenheit von Lösungs- und Verdünnungsmitteln und in Gegenwart eines Knoevenagel-Katalysators und/oder in Gegenwart einer reaktionsfähigen Erdalkaliverbindung bei einer Temperatur von 80 bis 160°C miteinander umsetzt, nach Abschluß der Additionsreaktion dem Reaktionsgemisch ein polares organisches Lösungsmittel, in dem sich das Additionsprodukt in der Wärme zumindest teilweise löst und dessen Siedepunkt oberhalb 240°C liegt,

zusetzt und die Mischung in Gegenwart von 1 bis 10 Mol% einer reaktionsfähigen Erdalkaliverbindung unter Abdestillieren der flüchtigen Pyrolyseprodukte auf 180 bis 250°C erwärmt. Zweckmäßigerweise wird das Lösungsmittel so gewählt, daß dessen Siedepunkt oberhalb der gewünschten Pyrolysetemperatur liegt.

Das erfindungsgemäße Verfahren wird ohne Isolierung eines Zwischenprodukts durchgeführt und kann daher in einem einzigen Reaktionsgefäß ausgeführt werden. Durch den Einsatz eines Knoevenagel-Katalysators kann die Addition von Thioglycolsäure an Acrylsäure, der erste Reaktionsschritt des erfindungsgemäßen Verfahrens, überraschenderweise ohne Lösungsmittel und sogar ohne jeden Acrylsäureüberschuß mit einer Ausbeute von 90 bis 95 % d. Th. durchgeführt werden. Selbstverständlich kann das Molverhältnis von Thioglycolsäure und Acrylsäure ohne Nachteile für die erhaltene Ausbeute auch im Rahmen der obigen Angaben (1 : 0,7 bis 1 : 1,5) variiert werden; diese Maßnahme bringt jedoch auch keine Vorteile, und jeder Überschuß von Acrylsäure muß nur wieder aus dem Reaktionsprodukt herausdestilliert werden. Eine weitere Erhöhung der Acrylsäuremenge verursacht zunehmend höhere Herstellkosten wegen der Wiedergewinnung und Reinigung des Acrylsäureüberschusses. Vorteilhafterweise wird daher bei einem Thioglycolsäure : Acrylsäure-Molverhältnis von 1 : 1 gearbeitet.

Knoevenagel-Katalysatoren werden zur Katalyse der Michael-Addition verwendet und sind bereits ausführlich beschrieben worden. (Z.B. H. Krauch, W. Kunz, "Reaktionen der organischen Chemie", 5. Auflage (1976), Seiten 77 ff.; Houben-Weyl, "Methoden der organischen Chemie", Band 4/II (1955), S. 25, 31; Band 5/1b (1972), S. 865, 866; Band 5/1c (1970), S. 519; Band 6/2 (1963), S. 690; Band 8 (1952), S. 450; G. Jones, "Organic Reactions", Band 15 (1967), S. 204.)

Für das erfindungsgemäße Verfahren können im Prinzip alle bekannten Knoevenagel-Katalysatoren eingesetzt werden. Vorwiegend aus Kostengründen ist es zweckmäßig, als Knoevenagel-Katalysator Ammoniak oder ein primäres oder sekundäres niederaliphatisches oder cyclisches Amin oder dessen Salz mit einer Carbonsäure, vorzugsweise einer niederaliphatischen Carbonsäure, einzusetzen.

Besonders bewährt haben sich als Knoevenagel-Katalysator Ammonacetat oder Piperidinacetat.

Der Knoevenagel-Katalysator kann in einer Menge von bis zu 5 Gew.%, bezogen auf das Gesamtgewicht der Reaktanten, eingesetzt werden. Vorzugsweise werden 0,01 bis 2,5 Gew.% eingesetzt.

Neben den oder anstelle der genannten Knoevenagel-Katalysatoren kann zur Katalyse der Addition von Thioglycolsäure an Acrylsäure in der ersten Reaktionsstufe des erfindungsgemäßen Verfahrens auch eine reaktionsfähige Erdalkaliverbindung eingesetzt werden. Reaktionsfähige Erdalkaliverbindungen im Sinne dieser Erfindung sind solche, die mit den Reaktanten Thioglycolsäure und Acrylsäure bei normaler oder erhöhter Temperatur in Reaktion treten z. B. unter Salzbildung. Reaktionsfähige Erdalkaliverbindungen sind somit Erdalkalisalze schwächerer organischer oder anorganischer Säuren wie z.B. die Erdalkaliacetate oder -carbonate sowie Erdalkalihydroxyde und -oxide. Vorzugsweise wird als reaktionsfähige Erdalkaliverbindung ein Erdalkalicarbonat oder ein Erdalkalihydroxyd, insbesondere Calcium- oder Bariumcarbonat oder -hydroxyd, eingesetzt.

Die Zusatzmenge der reaktionsfähigen Erdalkaliverbindung beträgt bis zu 10 Mol% der eingesetzten Acrylsäure, vorzugsweise 0,5 bis 5 Mol%.

Nach Abschluß der im ersten Reaktionsschritt vorgenommenen Addition von Thioglycolsäure an Acrylsäure wird in einem zweiten Reaktionsschritt das erhaltene Carboxymethyl-ßcarboxyethyl-sulfid (3-Thia-adipinsäure) unter Decarboxylierung zum gewünschten Tetrahydro-thiophen-3-on cyclisiert. Diese Reaktion erfolgt in Gegenwart eines polaren organischen Lösungsmittels, in welchem die 3-Thia-adipinsäure, zumindest in der Wärme, eine für die Reaktion ausreichende Löslichkeit aufweist, und das oberhalb von 240$^{o}$C siedet.

Die Löslichkeit der Thia-adipinsäure in dem einzusetzenden organischen Lösungsmittel sollte aus Gründen der Kleinhaltung des Reaktionsvolumens möglichst hoch sein und nicht unter 200 g/l liegen. Von dem organischen Lösungsmittel wird dem Ansatz so viel zugefügt, daß sich bei 180 bis 250$^{o}$C, der Cyclisierungstemperatur, mindestens 25% der Thia-adipinsäure, besser aber 50 bis 100 %, lösen.

Im Verlauf der Cyclisierungsreaktion destilliert das entstehende Tetrahydro-thiophen-3-on kontinuierlich ab, so daß sich, von Ausnahmefällen abgesehen, eine weitere Isolierungs- oder Reinigungsoperation erübrigt.

Nach dem Abdestillieren des Endprodukts hinterbleibt eine Lösung oder Suspension von hochsiedenden Nebenprodukten der Cyclisierungsreaktion in dem eingesetzen Lösungsmittel, aus der das Lösungsmittel durch Destillation, vorzugsweise Vakuumdestillation, leicht wieder regeneriert werden kann, die aber auch, gegebenenfalls unter Zusatz eines Anteils von frischem polaren organischen Lösungsmittel, erneut für einen folgenden Cyclisierungsansatz eingesetzt werden kann.

Besonders vorteilhaft kann man verfahren, wenn man jeweils 50 % des nach Abdestillieren des Tetrahydro-thiophen-3-ons erhaltenen Lösungsmittelrückstandes durch frisches Lösungsmittel ersetzt und die so erhaltene Mischung wieder für den folgenden Ansatz einsetzt. Die durch frisches Lösungsmittel ersetzten 50 % des Lösungsmittelrückstandes werden dann zur Regenerierung des reinen Lösungsmittels destilliert.

Polare organische Lösungsmittel, in denen der Cyclisierungsschritt des erfindungsgemäßen Verfahrens durchgeführt werden kann, sind insbesondere Sulfoxide und Sulfone mit einem Siedepunkt über etwa 240°C wie beispielsweise Sulfolan, Diethylsulfon, Phenyl-methyl-sulfoxid, Phenyl-ethyl-sulfoxid, Diphenyl-sulfoxid, 3,4-Dimethyl-tetrahydro-thiophen-1,1-dioxid, 3-Phenyl-tetrahydro-thiophen-1,1-dioxid.

Auch Gemische dieser Lösungsmittel untereinander und mit anderen hochsiedenden Lösungsmitteln wie z.B. hochsiedenden Kohlenwasserstoffen sind im erfindungsgemäßen Verfahren einsetzbar.

Besonders bewährt hat sich als polares organisches Lösungsmittel für das erfindungsgemäße Verfahren Sulfolan und als hochsiedender Kohlenwasserstoff das Handelsprodukt ®Marlotherm.

Die Ausbeute des zweiten Reaktionsschritts des erfindungsgemäßen Verfahrens beträgt 70 bis 80 % d. Th., so daß eine over-all-Ausbeute, das heißt Ausbeute bezogen auf die Ausgangsmaterialien Thioglycolsäure und Acrylsäure, von 66 - 76 % der Theorie erreicht wird.

Legt man die nach der Literatur belegten Ausbeutewerte für diesen Reaktionsweg zugrunde, nämlich 44 % für die Cyclisierung von 3-Thia-adipinsäure (Buiter, Recueil des Travaux Chimiques des Pays-Bas <u>83</u> (1964) S. 1161) und ca. 41 % für die Addition von Thioglycolsäure an Acrylsäure, so ergibt sich eine over-all-Ausbeute von nur 16 % der Theorie.

Zu der sehr beachtlichen und überraschenden Ausbeuteverbesserung kommen die Vereinfachung der Arbeitsweise durch das ununterbrochene Arbeiten ohne Isolierung von Zwischenprodukten und die einfache Durchführungsmöglichkeit in einem einzigen Reaktionsgefäß (Eintopfverfahren). Das erfindungsgemäße Verfahren ist somit für die Umsetzung in technische Größenordnungen bestens geeignet.

Ein wesentliches Merkmal des erfindungsgemäßen Verfahrens ist das Arbeiten ohne Zwischenisolierung der 3-Thia-adipinsäure. Es ist jedoch auch möglich, diese Verbindung nach der Durchführung des ersten Reaktionsschritts zu isolieren. Der erste Schritt des erfindungsgemäßen Verfahrens bietet nämlich gegenüber den bisher bekannten Methoden zur Herstellung von 3-Thia-adipinsäure den erheblichen Vorteil der schnellen und einfachen Durchführbarkeit und den der hohen Ausbeute. Somit ist dieser erste Schritt des erfindungsgemäßen Verfahrens auch als ein vorteilhafter Weg zur Herstellung von 3-Thia-adipinsäure geeignet. Die vorliegende Erfindung betrifft somit auch ein Verfahren zur Herstellung von 3-Thia-adipinsäure, bei dem man Thioglycolsäure mit Acrylsäure in Abwesenheit von Lösungs- oder Verdünnungsmitteln und in Gegenwart eines Knoevenagel-Katalysators und/-oder in Gegenwart einer reaktionsfähigen Erdalkaliverbindung bei einer Temperatur von 80 bis 160$^{\circ}$C miteinander umsetzt und nach Abschluß der Additionsreaktion das Reaktionsprodukt in an sich bekannter Weise vom Katalysator bzw. dem Erdalkalisalz befreit und gewünschtenfalls reinigt.

Auch der zweite Teilschritt des erfindungsgemäßen Verfahrens, der normalerweise unmittelbar an den ersten anschließt und die Cyclisierung von 3-Thia-adipinsäure zu Tetrahydro-thiophen-3-on beinhaltet, stellt für sich genommen eine sehr günstige Herstellungsmethode dieses Tetrahydro-thiophen-derivats aus 3-Thia-adipinsäure dar, die einfach und mit erheblich verbesserter Ausbeute durchzuführen ist.

Die vorliegende Erfindung betrifft somit auch ein Verfahren zur Herstellung von Tetrahydro-thiophen-3-on der Formel

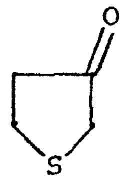

bei dem man beliebig hergestellte, gegebenenfalls auch mit organischen oder anorganischen Bestandteilen verunreinigte 3-Thia-adipinsäure in einem polaren organischen Lösungsmittel, dessen Siedepunkt oberhalb 240°C liegt, gegebenenfalls in der Wärme, zumindest teilweise löst und die Lösung in Gegenwart von bis zu 10 Mol% einer reaktionsfähigen Erdalkaliverbindung unter Abdestillieren der flüchtigen Pyrolyseprodukte auf 180 bis 250°C erwärmt, wobei zweckmäßigerweise das Lösungsmittel so gewählt wird, daß dessen Siedepunkt oberhalb der gewünschten Pyrolysetemperatur liegt.

Die folgenden Ausführungsbeispiele veranschaulichen die Durchführung des erfindungsgemäßen Verfahrens und seiner beiden Teilschritte.

<u>Beispiel 1:</u>

184 g (2 mol) Thioglycolsäure und 5 g (0,065 mol) Ammoniumacetat werden bei ca. 120°C vorgelegt. Bei einer Innentemperatur von ca. 120 - 130°C werden 144 g (2 mol) Acrylsäure zugetropft. Anschließend wird 1 Stunde bei ca. 120°C nachgerührt.

Dann werden 25 g (0,08 mol) Bariumhydroxid-octahydrat und 80 g Sulfolan zugesetzt. Die Temperatur wird auf ca. 240°C gesteigert. Unter gleichzeitiger $CO_2$-Entwicklung destillieren Wasser und Tetrahydro-thiophen-3-on ab. Im Destillat wird die untere organische Phase abgetrennt. Das erhaltene Rohprodukt kann für viele Umsetzungen direkt weiterverwendet werden.

Zur Herstellung von hochreinen Tetrahydrothiophen-3-on wird das Rohprodukt fraktioniert. Man erhält 155 g Produkt mit

einem Gehalt >99 % (GC), das entspricht 76 % d. Th. Kp.: 73 - 75°C/15 mm. Man erhält außerdem ca. 130 g flüssigen Pyrolyserückstand, der sich leicht wiederaufarbeiten läßt oder wiedereingesetzt werden kann (s. Beispiel 2).

Beispiel 2:

Man verfährt wie im Beispiel 1 angegeben, nur werden statt der 25 g Bariumhydroxid-octahydrat und 80 g Sulfolan die ca. 130 g Pyrolyserückstand zugesetzt. Man erhält das Produkt in praktisch identischer Ausbeute und Reinheit.

Beispiel 3:

184 g (2 mol) Thioglycolsäure und 25 g (0,08 mol) Bariumhydroxid-octahydrat werden bei ca. 120°C vorgelegt. Bei einer Innentemperatur von ca. 120 - 130°C werden 144 g (2 mol) Acrylsäure zugetropft. Anschließend wird 1 Stunde bei ca. 120°C gerührt. Dann werden 80 g Sulfolan zugesetzt. Es wird weiter verfahren wie im Beispiel 1 angegeben. Man erhält das Tetrahydro-thiophen-3-on in praktisch gleicher Ausbeute und Reinheit wie im Beispiel 1. Auch der hierbei erhaltene Pyrolyserückstand kann für Beispiel 2 eingesetzt werden.

Beispiel 4:

Man verfährt wie im Beispiel 1 oder 3 angegeben, nur wird statt Sulfolan die gleiche Menge Diethylsulfon verwendet. Man erhält das Tetrahydro-thiophen-3-on in praktisch identischer Ausbeute und Reinheit wie im Beispiel 1.

Beispiel 5:

Man verfährt wie im Beispiel 1 oder 3 angegeben, nur wird statt Sulfolan die gleiche Menge Phenyl-methyl-sulfoxid oder Phenyl-ethyl-sulfoxid verwendet.

Man erhält das Tetrahydro-thiophen-3-on in praktisch identischer Ausbeute und Reinheit wie im Beispiel 1.

Beispiel 6:

184 g (2 mol) Thioglycolsäure und 8 g (0,055 mol) Piperidinacetat werden bei ca. 120°C vorgelegt. Bei einer Innentemperatur von ca. 120 - 130°C werden 144 g (2 mol) Acrylsäure zugetropft. Anschließend wird 1 Stunde bei ca. 120°C nachgerührt.

Man erhält 336 g einer Rohschmelze von 3-Thia-adipinsäure mit einem Gehalt von 92,5 %, das entspricht 95 % d.Th.

Eine kristallisierte Probe hat einen Schmelzpunkt von 88 - 91°C. Bei Bedarf kann die erhaltene 3-Thiaadipinsäure weiter gereinigt werden.

Beispiel 7:

336 g einer nach Beispiel 6 hergestellten kristallisierten 3-Thia-adipinsäure werden aufgeschmolzen und bei 120°C vorgelegt. Dann werden 25 g (0,08 mol) Bariumhydroxid-octahydrat und 80 g Sulfolan zugesetzt. Die Temperatur wird auf ca. 240°C gesteigert. Unter gleichzeitiger $CO_2$-Entwicklung destillieren Wasser und Tetrahydro-thiophen-3-on ab.

Im Destillat wird die untere organische Phase abgetrennt. Das erhaltene Rohprodukt kann für viele Umsetzungen direkt weiterverwendet werden.

Zur Herstellung von hochreinem Tetrahydrothiophen-3-on wird das Rohprodukt fraktioniert. Man erhält 160 g Produkt mit einem Gehalt >99 % (GC), das entspricht 78 % d. Th.

Kp.: 73 - 75°C/15 mm.

Beispiel 8:

Vergleichsbeispiel nach Recueil des Travaux Chimiques des Pays-Bas 83 (1964), S. 1160 ff.

649 g (3,95 mol) 3-Thia-adipinsäure (Fp.: 90 - 92°C, hergestellt wie angegeben) und 30 g (0,18 mol) Bariumhydroxid-Pulver werden 4 Stunden auf ca. 230°C erhitzt. Es wird wie angegeben aufgearbeitet. Man erhält 170 g Tetrahydro--thiophen-3-on mit einem Gehalt von 97,5 % (GC), das entspricht 41 % d. Th.
Kp: 69 - 71°/12 mm.
Gleichzeitig fallen ca. 220 g eines dunklen, unangenehm riechenden, zähöligen bis festen teerartigen Rückstandes an, der sich nur unter großen Schwierigkeiten mechanisch aus dem Reaktionsgefäß entfernen läßt. Dieser Rückstand ist auch in dem in den Beispielen 1, 4, 5 angegebenen sowie in anderen Lösungsmitteln nur äußerst schlecht löslich.

PATENTANSPRÜCHE

1. Verfahren zur Herstellung von Tetrahydro-thiophen-3-on der Formel

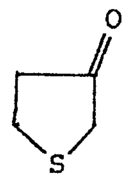

dadurch gekennzeichnet, daß man Thioglycolsäure mit Acrylsäure im Molverhältnis von 1 : 0,7 bis 1 : 1,5 in Abwesenheit von Lösungs- und Verdünnungsmitteln und in Gegenwart eines Knoevenagel-Katalysators und/oder in Gegenwart einer reaktionsfähigen Erdalkaliverbindung bei einer Temperatur von 80 bis 160°C miteinander umsetzt, nach Abschluß der Additionsreaktion dem Reaktionsgemisch ein polares organisches Lösungsmittel, in dem sich das Additionsprodukt in der Wärme zumindest teilweise löst und dessen Siedepunkt oberhalb 240°C liegt, zusetzt und die Mischung in Gegenwart von bis zu 10 Mol% einer reaktionsfähigen Erdalkaliverbindung unter Abdestillieren der flüchtigen Pyrolyseprodukte auf 180 bis 250°C erwärmt.

2. Verfahren gemäß Anspruch 1, dadurch gekennzeichnet, daß Thioglycolsäure und Acrylsäure im Molverhältnis von 1 : 1 miteinander umgesetzt werden.

3. Verfahren gemäß den Ansprüchen 1 und 2, dadurch gekennzeichnet, daß als Knoevenagel-Katalysator Ammoniak oder ein primäres oder sekundäres niederaliphatisches oder cyclisches Amin oder dessen Salz mit einer Carbonsäure, vorzugsweise einer niederaliphatischen Carbonsäure, eingesetzt wird.

4. Verfahren gemäß Anspruch 3, dadurch gekennzeichnet, daß als Knoevenagel-Katalysator Ammonacetat oder Piperidinacetat eingesetzt wird.

5. Verfahren gemäß den Ansprüchen 1 und 2, dadurch gekennzeichnet, daß die Umsetzung von Thioglycolsäure und Acrylsäure in Gegenwart einer reaktionsfähigen Erdalkaliverbindung durchgeführt wird.

6. Verfahren gemäß den Ansprüchen 1 bis 5, dadurch gekennzeichnet, daß als reaktionsfähige Erdalkaliverbindung ein Erdalkalicarbonat oder ein Erdalkalihydroxyd, vorzugsweise Calcium- oder Barium-carbonat oder -hydroxyd, eingesetzt wird.

7. Verfahren gemäß den Ansprüchen 1 bis 6, dadurch gekennzeichnet, daß man die Umsetzung in Gegenwart von bis zu 5 Gew.% eines Knoevenagel-Katalysators und/oder in Gegenwart von bis zu 10 Mol% einer reaktionsfähigen Erdalkaliverbindung durchführt.

8. Verfahren gemäß den Ansprüchen 1 bis 7, dadurch gekennzeichnet, daß als polares organisches Lösungsmittel Sulfolan eingesetzt wird.

9. Verfahren zur Herstellung von 3-Thia-adipinsäure, dadurch gekennzeichnet, daß man Thioglycolsäure mit Acrylsäure in Abwesenheit von Lösungs- oder Verdünnungsmitteln und in Gegenwart eines Knoevenagel-Katalysators und/oder in Gegenwart einer reaktionsfähigen Erdalkaliverbindung bei einer Temperatur von 80 bis 160$^oC$ miteinander umsetzt und nach Abschluß der Additionsreaktion das Reaktionsprodukt in an sich bekannter Weise vom Katalysator bzw. dem Erdalkalisalz befreit und gewünschtenfalls reinigt.

10. Verfahren zur Herstellung von Tetrahydro-thiophen-3-on der Formel

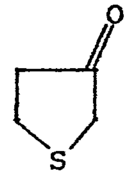

dadurch gekennzeiahnet, daß man 3-Thia-adipinsäure in einem polaren organischen Lösungsmittel, dessen Siedepunkt oberhalb 240°C liegt, gegebenenfalls in der Wärme, zumindest teilweise löst und die Lösung in Gegenwart einer reaktionsfähigen Erdalkaliverbindung unter Abdestillieren der flüchtigen Pyrolyseprodukte auf 180 bis 250°C erwärmt.

11. Tetrahydro-thiophen-3-on und 3-Thia-adipinsäure hergestellt nach dem Verfahren der Ansprüche 1 oder 9 bzw. 8.

Europäisches Patentamt

# EUROPÄISCHER RECHERCHENBERICHT

0127121
Nummer der Anmeldung

EP 84 10 5868

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | GB-A-2 122 185 (KODAK)<br>* Ansprüche * | 1 | C 07 D 333/32<br>C 07 C 149/20 |
| A | CH-A- 227 975 (HOFFMANN- LA ROCHE)<br>* Anspruch * | 1 | |

RECHERCHIERTE SACHGEBIETE (Int. Cl. 3)

C 07 D 333/00
C 07 C 149/00

Der vorliegende Recherchenbericht wurde für alle Patentansprüche erstellt.

| Recherchenort | Abschlußdatum der Recherche | Prüfer |
|---|---|---|
| DEN HAAG | 21-08-1984 | CHOULY J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82